# EUROPÄISCHE PATENTSCHRIFT

(11) **EP 1 845 079 B1**
(45) Veröffentlichungstag und Bekanntmachung des Hinweises auf die Patenterteilung: **26.03.2014**
(21) Anmeldenummer: 07075103.7
(22) Anmeldetag: 06.02.2007
(51) Int. Cl.: C07C 209/84, C07C 211/46, C07C 263/10, C07C 265/14, C07C 209/36, C07C 211/50, C07C 209/78

(54) **Verfahren zur Herstellung von Anilin**
Method for manufacturing aniline
Procédé de fabrication d'aniline

(30) Priorität: 18.02.2006 DE 102006007620
(43) Veröffentlichungstag der Anmeldung: 17.10.2007
(73) Patentinhaber: Bayer Intellectual Property GmbH, 40789 Monheim (DE)
(72) Erfinder: Dugal, Markus, 47906 Kempen (DE); Gehlen, Franz-Ulrich, 47839 Krefeld (DE); Wershofen, Stefan, 41065 Mönchengladbach (DE); Lago, Andre, 201107 Shanghai (CN); Lehner, Peter, 40878 Ratingen (DE); Marotz, Benie, 40227 Düsseldorf (DE)
(74) Vertreter: BIP Patents

(56) Entgegenhaltungen:
- US-A1- 2005 080 294
- DATABASE WPI Week 197439 Derwent Publications Ltd., London, GB; AN 1974-68661V XP002439053 -& JP 49 035341 A (SUMITOMO CHEM CO LTD) 1. April 1974 (1974-04-01)
- DATABASE WPI Week 199704 Derwent Publications Ltd., London, GB; AN 1997-037989 XP002439054 -& JP 08 295654 A (SUMITOMO CHEM CO LTD) 12. November 1996 (1996-11-12)

## Beschreibung

Die Erfindung betrifft ein Verfahren zur Herstellung von Anilin umfassend eine vereinfachte Aufreinigung von Anilin durch Destillation, bei dem Roh-Anilin vor der Destillation wässrige Alkalimetallhydroxid-Lösung zugegeben wird und/oder während der Destillation des Roh-Anilins wässrige Alkalimetallhydroxid-Lösung zugegeben wird, und dass der Sumpf aus der Destillation nach Zugabe der Alkalihydroxidlösung partiell ausgeschleust und mit Wasser oder verdünnter Alkalihydroxidlösung gewaschen wird, und die organische gewaschene Sumpfphase nach der Phasentrennung wieder in die Destillation zurückgeführt wird wodurch eine vereinfachte destillative Abtrennung phenolischer Komponenten aus dem Roh-Anilin ermöglicht wird.

Anilin ist wichtiges Zwischenprodukt z.B. zur Herstellung von Methylendiphenyldiisocyanat (MDI) und wird im großtechnischen Maßstab in der Regel durch katalytische Hydrierung von Nitrobenzol in der Gas- oder Flüssigphase hergestellt (siehe z.B. DE-OS 2201528, DE-OS 3414714, US-A-3136818, EP-A-0696573 und EP-A-0696574). Bei dieser Reaktion werden neben dem Zielprodukt Anilin auch Nebenkomponenten wie z.B. Phenol oder Aminophenole gebildet, die vor einer weiteren Anwendung des Anilins in Folgeprozessen durch Destillation entfernt werden müssen. Insbesondere die Trennung von Phenol und Anilin, stellt aufgrund der eng zusammen liegenden Siedepunkte für die Destillationstechnik eine große Herausforderung dar, was sich in der Verwendung von Destillationskolonnen mit hoher Trennleistung und hohen Rücklaufverhältnissen mit entsprechend hohem Investitions- und Energieaufwand widerspiegelt.

In JP-A-49-035341 wird ein alternatives Verfahren beschrieben, bei dem das Rohanilin mit festen Alkalien (z.B. festes Natriumhydroxid) in einem Festbett in Kontakt gebracht wird und erst anschließend in die Destillation geleitet wird, bzw. bei dem die Destillation in Gegenwart der festen Alkalie in Konzentrationen von 0,1-3 Gew.-% bezogen auf die zu destillierende Anilin-Menge durchgeführt wird. Hierdurch wird die Abtrennung farbkritischer Komponenten wie Aminophenole vereinfacht. Nachteilig bei diesem Verfahren ist jedoch der Einsatz von hohen molaren Überschüssen der festen Alkalien in Bezug auf die zu entfernenden sauren Nebenkomponenten bzw. die Unmöglichkeit der genauen Dosierung der alkalischen Verbindungen, was einerseits bei Überdosierung zu Korrosionsproblemen, Ausfällungen und hochviskosen Sumpfphasen in der Destillationskolonne und andererseits bei Unterdosierung zu einer unvollständigen Entfernung der kritischen Komponenten führen kann.

JP-A-08-295654 beschreibt als Alternative zur destillativen Entfernung von phenolischen Verbindungen aus Anilin eine Extraktion mit verdünnter wässriger Natronlauge (oder Kalilauge), wodurch das Phenol als Natriumphenolat großenteils in die wässrige Phase transferiert wird, die durch die anschließende Phasentrennung als obere Phase abgetrennt wird. Für eine effektive Reduzierung des Phenolgehaltes ist ein molares Verhältnis NaOH : Phenol im Bereich von 3:1 - 100:1 erforderlich. Nachteilig bei diesem Verfahren ist ein hoher NaOH-Verbrauch (molare Überschüsse), das Anfallen eines Na-phenolathaltigen Abwassers, was zu zusätzliche Entsorgungskosten führt sowie ein zusätzlicher Investitionsaufwand für die Extraktion.

US-A-2005/0080294 beschreibt ein Verfahren zur Herstellung von Anilin, in dem rohes Anilin enthaltend phenolische Verunreinigungen ggf. in Anwesenheit mehrfacher Alkohole wie Polyethylenglykol mit einer Base vermischt wird und die Mischung anschließend in einer Destillation gereinigt wird.

Die Erfindung betrifft ein Verfahren zur Herstellung von Anilin, bei dem
a) Roh-Anilin durch Hydrierung von Nitrobenzol in Gegenwart eines Katalysators hergestellt wird, und
b) das Roh-Anilin anschließend durch eine ein- oder mehrstufige Destillation aufgereinigt wird,
dadurch gekennzeichnet, dass dem Roh-Anilin vor der Destillation wässrige Alkalimetallhydroxid-Lösung zugegeben wird und/oder während der Destillation des Roh-Anilins wässrige Alkalimetallhydroxid-Lösung zugegeben wird, wobei die Menge der zugegebenen wässrigen Alkalimetallhydroxid-Lösung so eingestellt wird, dass das molare Verhältnis von Alkalimetallhydroxid zu dem in dem Roh-Anilin enthaltenen phenolischen Verbindungen in der Summe zwischen 0,1:1 und 10:1, bevorzugt zwischen 0,5:1 und 1,5:1, besonders bevorzugt zwischen 0,8:1 und 1,2:1 liegt, und dass der Sumpf aus der Destillation nach Zugabe der Alkalihydroxidlösung partiell ausgeschleust und mit Wasser oder verdünnter Alkalihydroxidlösung gewaschen wird, und die organische gewaschene Sumpfphase nach der Phasentrennung wieder in die Destillation zurückgeführt wird.

Unter phenolischen Verbindungen sind neben Phenol und Phenolat auch diejenigen Benzol-Derivate zusammengefasst, die zusätzlich zur OH-Funktion auch weitere funktionelle Gruppen tragen, wie. z.B. Aminophenole.

Das erfindungsgemäße Verfahren zeichnet sich aus durch eine vereinfachte Aufarbeitung des Roh-Anilins, bei dem durch die Zugabe von geringen Mengen wässriger Natronlauge, die bevorzugt unmittelbar vor dem Zulauf des Roh-Anilin-Stroms in die Destillation erfolgt, eine vereinfachte destillative Abtrennung phenolischer Komponenten ermöglicht wird.

Gegenüber dem in JP-A-49-035341 beschriebenen Stand der Technik bietet dieses Verfahren den Vorteil einer genauen stöchiometrischen oder gezielten über- oder unterstöchiometrischen Dosierung von Alkalimetallhydroxid in Bezug auf die im Anilin enthaltenen phenolischen Verbindungen was zu einer Vermeidung der angeführten Probleme der in JP-A-49-035341 beschriebenen Verfahrensweise führt. Gegenüber dem in JP-A-08-295654 beschriebenen Verfahren bietet das erfindungsgemäße Verfahren den Vorteil der Vermeidung von Phenolat-haltigen Abwässern, sowie der Vermeidung von zusätzlichen Extraktionsstufen mit Phasentrennung.

Bei dem erfindungsgemäßen Verfahren wird bei der Vereinigung der Alkalimetallhydroxid-Lösung mit dem Roh-Anilin vor der Destillation bzw. mit einem während der Destillation auftretenden Strom, beispielsweise einem flüssigen Anilin enthaltenden Strom, eine Lösung oder gegebenenfalls eine Emulsion erzeugt, in der vorhandene phenolische Verbindungen durch Reaktion mit Alkalimetallhydroxid in schwer siedende Phenolate überführt werden. Die Destillation und die Abtrennung der gebildeten Phenolate finden in dem erfindungsgemäßen Verfahren somit gleichzeitig in einem Schritt statt. Der Trennaufwand bzw. das Ergebnis der Trennung ist somit gegenüber den Verfahren des Standes der Technik optimiert.

Das erfindungsgemäße Verfahren umfasst die Hydrierung von Nitrobenzol in Schritt a). Darin können die technisch üblichen Verfahren zur Hydrierung von Nitrobenzol eingesetzt werden. Bevorzugt wird die Hydrierung von Nitrobenzol in der Gasphase an ortsfesten, heterogenen Trägerkatalysatoren, wie z.B. an Pd auf Aluminiumoxid oder auf Kohleträgern, in Festbettreaktoren bei einem absoluten Druck von 2 - 50 bar und einer Temperatur im Bereich von 250 - 500°C unter adiabatischen Bedingungen in Kreisgasfahrweise, d.h. unter Rückführung von in der Hydrierung nicht umgesetztem Wasserstoff, durchgeführt. Geeignete Verfahren sind beispielsweise in EP-A-0696573 und EP-A-0696574 offenbart.

Als geeignete wässrige Alkalimetallhydroxid-Lösungen werden in dem erfindungsgemäßen Verfahren bevorzugt Natrium- oder Kaliumhydroxid-Lösungen eingesetzt.

Die Konzentration an Alkalimetallhydroxid in der eingesetzten wässrigen Alkalimetallhydroxid-Lösung liegt bevorzugt zwischen 0,1 und 55 Gew.-%, besonders bevorzugt zwischen 1 und 50 Gew.-%, ganz besonders bevorzugt zwischen 3 und 35 Gew.-%, bezogen auf das Gewicht der wässrigen Alkalimetallhydroxid-Lösung.

Dabei wird durch eine zu hohe Konzentration an Alkalimetallhydroxid in der eingesetzten wässrigen Alkalimetallhydroxid-Lösung die Dosiergenauigkeit beeinträchtigt und durch eine zu niedrige Konzentration an Alkalimetallhydroxid in der eingesetzten wässrigen Alkalimetallhydroxid-Lösung der Wassereintrag in die Destillationskolonne erhöht, was zu einem erhöhten Energieverbrauch führt. Die zudosierte Menge an Alkalimetallhydroxid-Lösung richtet sich nach der im Rohanilin enthaltenen Konzentration an phenolischen Verbindungen, welche durch z.B. gaschromatographische Methoden bestimmt werden kann, und nach der Konzentration der Alkalimetallhydroxid-Lösung. Bevorzugt wird die zugegebene bzw. zudosierte Menge an wässriger Alkalimetallhydroxid-Lösung so eingestellt, dass die molare Menge der im Roh-Anilin enthaltenen phenolischen Verbindungen der molaren Menge des in der wässrigen Alkalimetallhydroxid-Lösung enthaltenen Alkalimetallhydroxids entspricht oder ein geringfügiger Unterschuss an Alkalimetallhydroxid vorliegt. Die Stöchiometrie der Zugabe der wässrigen Alkalimetallhydroxid-Lösung kann aber auch gezielt in Richtung eines molaren Überschusses oder deutlicheren molaren Unterschusses an Alkalimetallhydroxid gegenüber phenolischen Verbindungen angepasst werden.

Die Zugabe der Alkalimetallhydroxid-Lösung in dem erfindungsgemäßen Verfahren erfolgt zum Roh-Anilin vor der Destillation und/oder während der Destillation des Roh-Anilins. Bevorzugt erfolgt die Zugabe der Alkalimetallhydroxid-Lösung zum Roh-Anilin unmittelbar (d.h. üblicherweise nicht mehr als 15 min) vor der Destillation. Sie kann aber auch während der Destillation, beispielsweise in einen flüssigen Anilin enthaltenden Strom zwischen zwei Destillationsstufen oder in einen flüssigen Anilin enthaltenden Rücklauf erfolgen. Bevorzugt erfolgt die Zugabe der Alkalimetallhydroxid-Lösung über eine Dosierpumpe, bei der die Durchflussmenge manuell oder über eine automatische Regelung eingestellt werden kann. Die Vermischung der Alkalimetallhydroxid-Lösung mit dem der Destillation zulaufenden Roh-Anilin oder einen während der Destillation auftretenden Anilin enthaltenden Strom kann auf verschiedene Arten erfolgen, z.B. durch statische oder dynamische Mischelemente, Rührkessel, Pumpen oder durch natürliche Konvektion.

Alternativ oder zusätzlich zu der Zugabe der Alkalimetallhydroxidlösung zum Zulauf der Destillationskolonne kann eine Zudosierung von Alkalimetallhydroxidlösung auch im Sumpf bzw. Sumpfumlauf oder in den Rücklaufströmen der Kolonne erfolgen.

Die destillative Aufarbeitung des Gemischs aus Alkalimetallhydroxid-Lösung und Roh-Anilin kann nach verschiedenen Methoden unter Einstellung einer großen Bandbreite an Bedingungen erfolgen. Dabei kann die Destillation ein- oder mehrstufig in den verschiedenen Kolonnentypen, bevorzugt in konventionellen Rektifikationskolonnen oder in Ausführungen als Trennwandkolonnen und mit verschiedenen Einbauten wie z.B. Sieb-, Ventil- oder auch Glockenböden, Füllkörpern oder Packungen erfolgen. Auch andere Ausführungsformen sind möglich. Bevorzugt erfolgt die Destillation in einer Destillationskolonne oder in zwei oder in drei seriell verschalteten Destillationskolonnen. Die Betriebsparameter Kopfdruck und Rücklaufverhältnis sind immer in Abhängigkeit von der Zusammensetzung des Roh-Anilins, der Spezifikation/Reinheit des gewünschten gereinigten Anilins (Rein-Anilins) und den zur Verfügung stehenden Trennstufen zu wählen. Die Abtrennung von Leichtsiedern, wie z.B. Wasser, Benzol, Cyclohexan, Cyclohexylamin, Cyclohexanon und Schwersiedern wie z.B. Phenol, Alkaliphenolate, Aminophenole, Alkaliaminophenolate, Phenylendiamine, Diphenylamin etc. kann dabei separat in verschiedenen Kolonnen erfolgen oder alternativ in einer bevorzugten Ausführungsform kombiniert in einer Kolonne unter Kopfentnahme der Leichtsieder, Sumpfentnahme der Schwersieder und Seitenstromentnahme des Reinanilins. Die destillative Reinigung des Roh-Anilins erfolgt in einer bevorzugten Ausführungsform in einer Seitenstromkolonne, besonders bevorzugt in einer Trennwandkolonne, unter Entnahme der Leichtsieder am Kopf, Entnahme der Schwersieder am Sumpf und Entnahme des Rein-Anilins im Seitenstrom. Weiterhin kann das Sumpfprodukt der Schwersiederabtrennung optional in einer Reststoffkolonne weiter eingeengt werden, um den Anilin-Verlust zu minimieren.

Aus der Reststoffkolonne abgetrenntes Anilin wird dabei in der Regel in die Hauptdestillation zurückgeführt. Die aufkonzentrierte Sumpfphase wird in der Regel einer Verbrennung zugeführt.

Der Sumpf aus der Destillationskolonne wird nach der Zugabe der Alkalihydroxidlösung partiell ausgeschleust und mit Wasser oder verdünnter Alkalihydroxidlösung gewaschen, um die Konzentration von Phenolat-Salzen im Kolonnensumpf abzusenken und die Sumpfphase in der Destillation stärker aufkonzentrieren zu können (geringerer Produktverlust), ohne dass Probleme durch Fouling, Feststoffabscheidung oder starken Viskositätsanstieg auftreten. Die Wäsche kann dabei in beliebigen dafür geeigneten Apparaturen wie z.B. Mixer-Settler-Apparaturen, Extraktionskolonnen oder einer Kombination aus statischen Mischelementen mit nachgeschalteten Phasentrennern erfolgen. Das zur Wäsche verwendete Wasser kann dabei einer beliebigen Quelle entstammen. Bevorzugt wird zur Minimierung des Abwasserstroms Prozesswasser verwendet, welches in der vorgeschalteten Reaktion entsteht. Die Wäsche wird ein- oder mehrstufig bei beliebig wählbaren Phasenverhältnissen und Temperaturen durchgeführt, bevorzugt im Bereich von 10 °C - 150 °C. Die organische gewaschene Sumpfphase wird nach der Phasentrennung wieder an einer beliebigen Stelle in die Destillationskolonne zurückgeführt, bevorzugt aber direkt in den Sumpfkreislauf. Die phenolathaltige wässrige Phase aus der Sumpfwäsche, kann anschließend einer Kläranlage zur Entsorgung zugeführt werden, wobei optional zuvor das Abwasser in einer Strippkolonne weitgehend von Anilin-Resten befreit werden kann. Alternativ kann die wässrige Phase aus der Sumpfwäsche direkt oder nach Aufkonzentrierung durch Verdampfen von Wasser einer Verbrennung zugeführt werden.

Das so erhältliche gereinigte Anilin enthält bevorzugt weniger als 0,01 Gew.-%, besonders bevorzugt weniger als 0,005 Gew.-% an phenolischen Verbindungen in der Summe, bezogen auf das Gewicht des Anilins.

Der Zulauf des Anilins und der Alkalimetallhydroxid-Lösung in die Destillationskolonne, kann an einer beliebigen Stelle der Kolonne erfolgen, bevorzugt erfolgt der Zulauf jedoch in Abhängigkeit vom Konzentrationsprofil an Anilin in der Destillationskolonne in der Kolonnenmitte oder in der unteren Kolonnenhälfte. Die Kolonne kann einen Abtriebs- und/oder einen Verstärkerteil besitzen. Die Zulauftemperatur in die Kolonne, sowie die Sumpftemperatur, Kopfdruck und Rücklaufverhältnis sind einstellbar und können der Trennaufgabe, sowie den qualitativen, betrieblichen und ökonomischen Erfordernissen angepasst werden. Die Temperatur am Kopf der Kolonne stellt sich entsprechend der gewählten Voreinstellungen der genannten Parameter und der Zusammensetzung der flüssigen Phase und der Dampfphase in der Kolonne ein. Bevorzugte Bedingungen für Betriebsparameter der Destillationskolonne bzw. Destillationskolonnen sind absolute Drücke von 10 - 1000 mbar, besonders bevorzugt von 10-500 mbar und Rücklaufverhältnisse von 0,1 - 3, ganz besonders bevorzugt von 0,3 - 0,8.

In einer bevorzugten Ausführungsform des erfindungsgemäßen Verfahrens erfolgt die Zudosierung der Alkalimetallhydroxid-Lösung in den Zulauf des Roh-Anilins einer Leichtsiederkolonne, bei der die Leichtsieder und Wassers über Kopf der Kolonne abgetrennt werden. Das im Sumpf anfallende Gemisch enthaltend Anilin und Alkalimetallhydroxid wird anschließend einem weiteren Destillationsschritt (Schwersiederabtrennung oder Reindestillation) zugeführt. Optional folgt schließlich eine Aufkonzentrierung des Sumpfes aus der Schwersiederabtrennung oder Reindestillation in einer Reststoffkolonne, wobei das in der Reststoffkolonne über Kopf gewonnene Anilin wieder in die Kolonne der Schwersiederabtrennung oder Reindestillation oder in die Leichtsiederkolonne bzw. einer vorgeschalteten Phasentrennung zurückgeführt werden kann.

In einer weiteren bevorzugten Ausführungsform erfolgt der Zulauf des Anilins und der Alkalimetallhydroxid-Lösung in eine kombinierte Leichtsieder- und Schwersiederkolonne (Seitenstromkolonne), wobei die Leichtsieder über Kopf, die Schwersieder als Sumpfphase und das Reinanilin als Seitenstrom abgeführt wird. Diese Seitenstromkolonne ist sowohl als konventionelle Kolonne (d.h. ohne Trennwand) als auch als Trennwandkolonne realisierbar. Diese Ausführungsform, bei der eine Seitenstromkolonne oder Trennwandkolonne eingesetzt wird, erfordert eine Phasentrennung der am Kopf entnommenen kondensierten Brüden, die im Wesentlichen das Azeotrop Wasser/Anilin und Leichtsieder enthalten. Wasser und in der wässrigen Phase gelöste Leichtsieder werden bevorzugt abgeführt, das Anilin wird bevorzugt zur Kolonne zurückgeführt.

Bevorzugt werden in dieser Ausführungsform des erfindungsgemäßen Verfahrens die am Kopf der Seitenstromkolonne entnommen Brüden in einer zweistufigen Kondensation kondensiert. Dabei kondensiert der erste Kondensator bevorzugt partiell die schwerer siedenden Komponenten der Brüden. In dem zweiten, nachgeschalteten Kondensator werden bevorzugt die durchgeschlagenen Leichtsieder kondensiert, die somit separat ausgeschleust werden können. Das Partialkondensat aus dem ersten Kondensator wird einer Phasentrennung zugeführt. Wasser und in der wässrigen Phase gelöste Leichtsieder werden bevorzugt abgeführt, das Anilin wird bevorzugt zur Kolonne zurückgeführt.

Das im Seitenstrom entnommene Rein-Anilin wird bevorzugt partiell als Rücklauf auf die Seitenstromkolonne unterhalb der Entnahmestelle des Seitenstroms aufgegeben. Die Seitenstromentnahme kann als Totalentnahme oder als partielle Entnahme realisiert werden. In beiden Fällen ist eine gezielte Einstellung des Rücklaufverhältnisses realisierbar.

In einer weiteren besonderen Ausführungsform des erfindungsgemäßen Verfahrens können auch eine oder mehrere Destillationsstufen oder andere Aufarbeitungsschritte der Dosierung der Alkalimetallhydroxid-Lösung zum Roh-Anilin vorgeschaltet werden. So kann z.B. vor der Zugabe der Alkalimetallhydroxidlösung eine destillative Leichtsiederabtrennung erfolgen. Nach der Dosierung kann dann die Schwersiederabtrennung in einer nachgeschalteten Reinkolonne mit Entnahme des Rein-Anilins als Kopfprodukt und optional eine Aufkonzentrierung des Sumpfes in einer weiteren Destillation (Reststoffkolonne) erfolgen.

Das nach dem erfindungsgemäßen Verfahren erhaltene Anilin kann anschließend nach den aus dem Stand der Technik bekannten Verfahren mit Formaldehyd in Gegenwart eines sauren Katalysators zu Di- und Polyaminen der Diphenylmethanreihe umgesetzt werden. Die Di- und Polyaminen können anschließend nach den aus dem Stand der Technik bekannten Verfahren mit Phosgen zu den entsprechenden Di- und Polyisocyanaten der Diphenylmethareihe umgesetzt werden.

In den folgenden Beispielen werden Varianten der Durchführung aufgezeigt.

### Allgemeine Vorbemerkung zur Versuchsdurchführung:

Die nachfolgenden Beispiele werden unter stationären Betriebsbedingungen in einer Miniplant Laboranlage durchgeführt.

Die Vereinigung und Durchmischung des Roh-Anilins mit der Alkalimetallhydroxid-Lösung erfolgt in den Beispielen 1, 2, 3 und 5 vor der Einspeisung in die Destillationskolonne. In Beispiel 4 wird die Alkalimetallhydroxid-Lösung in den Rücklauf des Seitenstroms der Kolonne zugegeben. Die Alkalimetallhydroxid-Lösung kann in verschiedenen, definierten Konzentrationen in Abhängigkeit von der Phenolkonzentration des Roh-Anilins und der angestrebten Abreicherung unter Berücksichtigung der als Kopfprodukt zu erwartenden Wassermenge dosiert werden. Die der Destillation zugeführte Wassermenge in Form von Alkalimetallhydroxid-Lösung hat direkten Einfluss auf den benötigten Energieverbrauch und somit auf die Wirtschaftlichkeit des Verfahrens. Weiterhin ändert sich je nach Konzentration an Wasser im Zulauf auch die Hydrodynamik und somit ggf. die Trennleistung der verwendeten Kolonneneinbauten.

Das verwendete Roh-Anilin enthält folgende Schlüsselkomponenten in den Konzentrationsbereichen (bezogen auf Gewicht) wie folgt:

| | |
|---|---|
| Benzol | 100 -2000 ppm |
| Anilin | 99,5-99,9 Gew.-% |
| Phenol | 200 - 1000 ppm |
| Diphenylamin | 40 - 1500 ppm |

Unter Berücksichtigung der oben angegebenen Randbedingungen und der gewünschten Phenolabreicherung werden die nachfolgenden, Versuche durchgeführt.

### Beispiel 1

Zu Roh-Anilin werden 32 Gew.-% NaOH-Lösung in einem molaren Verhältnis Phenol zu NaOH von 1 : 1,3 zudosiert. Die Mischung der beiden Flüssigkeiten erfolgt in einem statischen Mischer. Die Kolonne wird bei einem absoluten Druck von 133 mbar und einem Rücklaufverhältnis R/E = 0,9 unter stationären Betriebsbedingungen betrieben.

Das eingesetzte Roh-Anilin enthält die Schlüsselkomponenten in folgenden Konzentrationen

| | Gew.-% |
|---|---|
| Benzol | 0,0225% |
| Anilin | 99,9314% |
| Phenol | 0,0252% |
| Diphenylamin | 0,0040% |

Nach der direkten Zudosierung der NaOH-Lösung in das Roh-Anilin und der Mischung im Statikmischer und der anschließenden Destillation wird im Seitenstrom das Produkt mit folgenden Konzentrationen entnommen:

| | Gew.-% |
|---|---|
| Benzol | 0,0002% |
| Anilin | 99,9975% |
| Phenol | 0,0009% |
| Diphenylamin | 0,0000% |

### Beispiel 2

Zu Roh-Anilin werden 32 Gew.-% NaOH-Lösung in einem molaren Verhältnis Phenol zu NaOH von 1 : 1,4 zudosiert. Die Mischung der beiden Flüssigkeiten erfolgt durch eine Zahnradpumpe. Die Kolonne wird bei einem absoluten Druck von 133 mbar und einem Rücklaufverhältnis R/E = 0,7 unter stationären Betriebsbedingungen betrieben.

Das eingesetzte Roh-Anilin enthält die Schlüsselkomponenten in folgenden Konzentrationen:

| | Gew.-% |
|---|---|
| Benzol | 0,0550% |
| Anilin | 99,8299% |
| Phenol | 0,0556% |
| Diphenylamin | 0,0406% |

Nach der direkten Zudosierung der NaOH-Lösung in das Roh-Anilin und der Mischung der beiden Flüssigkeiten in der Pumpe weist das Roh-Anilin folgende Konzentrationen der Schlüsselkomponenten auf:

| | Gew.-% |
|---|---|
| Benzol | 0,0519% |
| Anilin | 99,8808% |
| Phenol | 0,0068% |
| Diphenylamin | 0,0425% |

In der GC-Analyse wird eine geringere Phenol-Konzentration festegestellt, was auf die Umwandlung von Phenol mit NaOH in hochsiedendes (nicht GC-gängiges) Na-Phenolat zurückzuführen ist.

Nach der destillativen Trennung des Roh-Anilins in Leichtsieder (Kopfprodukt), Hochsieder (Sumpfprodukt) und Mittelsieder (Seitenstrom) weist das als Seitenstrom entnommene Rein-Anilin folgende Konzentrationen der Schlüsselkomponenten auf:

| | Gew.-% |
|---|---|
| Benzol | 0,0011% |
| Anilin | 99,9942% |
| Phenol | 0,0006% |
| Diphenylamin | 0,0000% . |

### Beispiel 3

Zu Roh-Anilin werden 50 Gew.-% NaOH-Lösung in einem molaren Verhältnis Phenol zu NaOH von 1 : 1 zudosiert. Die Mischung der beiden Flüssigkeiten erfolgt durch eine Zahnradpumpe. Die Kolonne wird bei einem absoluten Druck von 133 mbar und einem Rücklaufverhältnis R/E = 0,8 unter stationären Betriebsbedingungen betrieben.

Das eingesetzte Roh-Anilin enthält die Schlüsselkomponenten in folgenden Konzentrationen:

| | Gew.-% |
|---|---|
| Benzol | 0,0550% |
| Anilin | 99,8299% |
| Phenol | 0,0556% |
| Diphenylamin | 0,0406% |

Nach der direkten Zudosierung der NaOH-Lösung in das Roh-Anilin und der Mischung der beiden Flüssigkeiten in der Pumpe und der anschließenden Destillation weist das Roh-Anilin folgende Konzentrationen der Schlüsselkomponenten auf:

| | Gew.-% |
|---|---|
| Benzol | 0,0002% |
| Anilin | 99,9975% |
| Phenol | 0,0009% |
| Diphenylamin | 0,0000% |

### Beispiel 4

Zu dem Seitenstromrücklauf der Destillation von Roh-Anilin in einer Seitenstromkolonne werden 32 Gew.-% NaOH-Lösung in einem molaren Verhältnis Phenol (im Zulauf Roh-Anilin) zu NaOH von 1 : 1,4 zudosiert. Die Mischung der beiden Flüssigkeiten erfolgt durch Konvektion. Die Kolonne wird bei einem absoluten Druck von 133 mbar und einem Rücklaufverhältnis R/E = 0,8 unter stationären Betriebsbedingungen betrieben.

Das eingesetzte Roh-Anilin enthält die Schlüsselkomponenten in folgenden Konzentrationen

| | Gew.-% |
|---|---|
| Benzol | 0,0502% |
| Anilin | 99,8347% |
| Phenol | 0,0553% |
| Diphenylamin | 0,0410% |

Das im stationären Betrieb unter Zudosierung der NaOH-Lösung in den Seitenstromrücklauf, Mischung der NaOH-Lösung mit dem Seitenstromrücklauf (Rein-Anilin) und Destillation erhaltene Seitenstromprodukt (Rein-Anilin) enthält die Schlüsselkomponenten in den folgenden Konzentrationen:

| | Gew.-% |
|---|---|
| Benzol | 0,0007% |
| Anilin | 99,9921% |
| Phenol | 0,0028% |
| Diphenylamin | 0,0000% . |

### Beispiel 5

Zu Roh-Anilin werden 5 Gew.-% NaOH-Lösung in einem molaren Verhältnis Phenol zu NaOH von 1 : 0,95 zudosiert. Die Mischung der beiden Flüssigkeiten erfolgt durch eine Zahnradpumpe. Die Kolonne wird bei einem absoluten Druck von 133 mbar und einem Rücklaufverhältnis R/E = 0,8 unter stationären Betriebsbedingungen betrieben.

Das eingesetzte Roh-Anilin enthält die Schlüsselkomponenten in folgenden Konzentrationen:

| | Gew.-% |
|---|---|
| Benzol | 0,0902% |
| Anilin | 99,7041% |
| Phenol | 0,0440% |
| Diphenylamin | 0,1359% |

Nach der direkten Zudosierung der NaOH-Lösung in das Roh-Anilin und der Mischung der beiden Flüssigkeiten in der Pumpe und der anschließenden destillativen Trennung des Roh-Anilins in Leichtsieder (Kopfprodukt), Hochsieder (Sumpfprodukt) und Mittelsieder (Seitenstrom) weist das als Seitenstrom entnommene Rein-Anilin folgende Konzentrationen der Schlüsselkomponenten auf:

| | Gew.-% |
|---|---|
| Benzol | 0,0015% |
| Anilin | 99,9887% |
| Phenol | 0,0038% |
| Diphenylamin | 0,0000% |

## Patentansprüche

1. Verfahren zur Herstellung von Anilin, bei dem
a) Roh-Anilin durch Hydrierung von Nitrobenzol in Gegenwart eines Katalysators hergestellt wird, und
b) das Roh-Anilin anschließend durch eine ein- oder mehrstufige Destillation aufgereinigt wird,
**dadurch gekennzeichnet, dass** dem Roh-Anilin vor der Destillation wässrige Alkalimetallhydroxid-Lösung zugegeben wird und / oder während der Destillation des Roh-Anilins wässrige Alkalimetallhydroxid-Lösung zugegeben wird, wobei die Menge der zugegebenen wässrigen Alkalimetallhydroxid-Lösung so eingestellt wird, dass das molare Verhältnis von Alkalimetallhydroxid zu dem in dem Roh-Anilin enthaltenen phenolischen Verbindungen in der Summe zwischen 0,1 und 10:1 liegt, und dass der Sumpf aus der Destillation nach Zugabe der Alkalihydroxidlösung partiell ausgeschleust und mit Wasser oder verdünnter Alkalihydroxidlösung gewaschen wird, und die organische gewaschene Sumpfphase nach der Phasentrennung wieder in die Destillation zurückgeführt wird.

2. Verfahren nach Anspruch 1 bei dem die Hydrierung des Nitrobenzols in der Gasphase unter adiabatischen Bedingungen in Festbettreaktoren in Gegenwart eines Pd-haltigen Katalysators und unter Rückführung von in der Hydrierung nicht umgesetztem Wasserstoff durchgeführt wird.

3. Verfahren nach einem der Ansprüche 1 oder 2, bei dem als Alkalimetallhydroxid-Lösung Natrium- und / oder Kaliumhydroxid-Lösung eingesetzt wird.

4. Verfahren nach einem der Ansprüche 1 bis 3, bei dem die Alkalimetallhydroxid-Lösung das Alkalimetallhydroxid in Konzentrationen von zwischen 0,1 und 55 Gew.-%, bezogen auf das Gewicht der wässrigen Alkalimetallhydroxid-Lösung, enthält.

5. Verfahren nach einem der Ansprüche 1 bis 4, bei dem die Alkalimetallhydroxid-Lösung mit dem Roh-Anilin vor der Destillation durch statische oder dynamische Mischelemente vermischt wird.

6. Verfahren nach einem der Ansprüche 1 bis 5, bei dem die Destillation einstufig in einer Seitenstromkolonne durchgeführt wird, wobei die Leichtsieder über Kopf, die Schwersieder als Sumpfprodukt und das gereinigte Anilin als Seitenstrom abgeführt wird.

7. Verfahren nach einem der Ansprüche 1 bis 5 bei dem die Destillation einstufig in einer Trennwandkolonne durchgeführt wird, wobei die Leichtsieder über Kopf, die Schwersieder als Sumpfprodukt und das gereinigte Anilin als Seitenstrom abgeführt wird.

8. Verfahren nach einem der Ansprüche 6 oder 7, bei dem die in der Seitenstromkolonne oder Trennwandkolonne am Kopf abgezogenen Brüden in einer zweistufigen Kondensation kondensiert werden.

9. Verfahren nach einem der Ansprüche 1 bis 5, bei dem die Destillation zunächst in einer Leichtsiederkolonne erfolgt, wobei die Leichtsieder und Wasser am Kopf der Leichtsiederkolonne abgezogen werden und das im Sumpf anfallende Gemisch enthaltend Anilin und Alkalimetallhydroxid anschließend in einem weiteren Destillationsschritt weiter aufgetrennt wird.

10. Verfahren nach einem der Ansprüche 1 bis 5, bei dem aus dem Roh-Anilin vor der Zugabe der Alkalimetallhydroxid-Lösung destillativ Leichtsieder entfernt werden und bei dem nach der Zugabe der Alkalimetallhydroxid-Lösung aus dem Roh-Anilin Schwersieder destillativ abgetrennt werden, wobei das gereinigte Anilin als Kopfprodukt und Schwersieder als Sumpfprodukt erhalten wird.

11. Verfahren nach Anspruch 10, bei dem die als Sumpfprodukt erhaltenen Schwersieder anschließend in einer Destillationskolonne aufkonzentriert werden.

12. Verfahren zur Herstellung von Di- und Polyaminen der Diphenylmethanreihe, bei dem man Anilin nach einem Verfahren gemäß Anspruch 1 herstellt und anschließend das Anilin mit Formaldehyd in Gegenwart eines sauren Katalysators zu den Di- und Polyaminen umsetzt.

13. Verfahren zur Herstellung von Di- und Polyisocyanaten der Diphenylmethanreihe, bei dem man Anilin nach einem Verfahren gemäß Anspruch 1 herstellt und anschließend das Anilin mit Formaldehyd in Gegenwart eines sauren Katalysators zu Di- und Polyaminen umsetzt und anschließend die Di- und Polyamine mit Phosgen zu den Di- und Polyisocyanaten umsetzt.

## Claims

1. Process for preparing aniline, comprising
a) preparing crude aniline by hydrogenation of nitrobenzene in the presence of a catalyst and
b) subsequently purifying the crude aniline by single- or multi-stage distillation,
**characterized in that** aqueous alkali metal hydroxide solution is added to the crude aniline prior to the distillation and/or aqueous alkali metal hydroxide solution is added during the distillation of the crude aniline, the amount of the added aqueous alkali metal hydroxide solution being adjusted such that the molar ratio of alkali metal hydroxide to total phenolic compounds comprised in the crude aniline is between 0.1 and 10 : 1, and **in that**, following addition of the alkali metal hydroxide solution, the bottoms from the distillation are partially removed and washed with water or dilute alkali metal hydroxide solution and the washed organic bottom phase is recycled into the distillation following phase separation.

2. Process according to Claim 1, wherein the hydrogenation of the nitrobenzene is carried out in the gas phase under adiabatic conditions in fixed-bed reactors in the presence of a Pd-containing catalyst and with recycling of hydrogen that has not reacted in the hydrogenation.

3. Process according to either of Claims 1 and 2, wherein sodium hydroxide solution and/or potassium hydroxide solution are used as the alkali metal hydroxide solution.

4. Process according to any one of Claims 1 to 3, wherein the alkali metal hydroxide solution comprises the alkali metal hydroxide in concentrations of between 0.1 and 55 wt% based on the weight of the aqueous alkali metal hydroxide solution.

5. Process according to any one of Claims 1 to 4, wherein the alkali metal hydroxide solution is mixed with the crude aniline prior to the distillation, using static or dynamic mixing elements.

6. Process according to any one of Claims 1 to 5, wherein the distillation is carried out in a single stage in a side stream column, the low boilers being removed overhead, the high boilers being removed as bottom product and the purified aniline being removed as a side stream.

7. Process according to any one of Claims 1 to 5, wherein the distillation is carried out in a single stage in a dividing wall column, the low boilers being removed overhead, the high boilers being removed as bottom product and the purified aniline being removed as a side stream.

8. Process according to either of Claims 6 and 7, wherein the vapours withdrawn overhead in the side stream column or the dividing wall column are condensed in a two-stage condensation.

9. Process according to any one of Claims 1 to 5, wherein the distillation is initially carried out in a low-boiler column, the low boilers and water being withdrawn at the top of the low boiler column and the aniline- and alkali metal hydroxide-containing mixture formed at the bottom subsequently being further separated in an additional distillation step.

10. Process according to any one of Claims 1 to 5, wherein low boilers are distillationly removed from the crude aniline prior to the addition of alkali metal hydroxide solution and wherein high boilers are distillationly separated from the crude aniline following the addition of the alkali metal hydroxide solution, the purified aniline being obtained as head product and low boilers being obtained as bottom product.

11. Process according to Claim 10, wherein the high boilers obtained as bottom product are subsequently concentrated in a distillation column.

12. Process for preparing di- and polyamines of the diphenylmethane series wherein aniline is prepared by a process according to Claim 1 and the aniline is subsequently reacted with formaldehyde in the presence of an acidic catalyst to form the di- and polyamines.

13. Process for preparing di- and polyisocyanates of the diphenylmethane series wherein aniline is prepared by a process according to Claim 1 and the aniline is subsequently reacted with formaldehyde in the presence of an acidic catalyst to form di- and polyamines and the di- and polyamines are subsequently reacted with phosgene to form the di- and polyisocyanates.

## Revendications

1. Procédé pour la production d'aniline, dans lequel
a) on prépare de l'aniline brute par hydrogénation de nitrobenzène en présence d'un catalyseur, et
b) ensuite on purifie l'aniline brute par une distillation en un ou plusieurs stades,
**caractérisé en ce qu'**avant la distillation on ajoute à l'aniline brute une solution aqueuse d'un hydroxyde de métal alcalin et/ou pendant la distillation de l'aniline brute on ajoute une solution aqueuse d'un hydroxyde de métal alcalin, en ajustant la quantité de la solution aqueuse d'hydroxyde de métal alcalin ajoutée de manière que le rapport molaire de l'hydroxyde de métal alcalin aux composés phénoliques contenus au total dans l'aniline brute soit compris entre 0,1 et 10:1, et **en ce que** le résidu de la distillation après addition de la solution d'hydroxyde de métal alcalin est partiellement évacué et lavé avec de l'eau ou une solution diluée d'hydroxyde de métal alcalin, et après la séparation de phases la phase résiduelle organique lavée est de nouveau renvoyée dans la distillation.

2. Procédé selon la revendication 1, dans lequel l'hydrogénation du nitrobenzène dans la phase gazeuse est effectuée dans des conditions adiabatiques, dans des réacteurs à lit fixe, en présence d'un catalyseur contenant du Pd et avec recyclage de l'hydrogène n'ayant pas réagi dans l'hydrogénation.

3. Procédé selon l'une quelconque des revendications 1 et 2, dans lequel on utilise comme solution d'hydroxyde de métal alcalin une solution d'hydroxyde de sodium et/ou de potassium.

4. Procédé selon l'une quelconque des revendications 1 à 3, dans lequel la solution d'hydroxyde de métal alcalin contient l'hydroxyde de métal alcalin à des concentrations comprises entre 0,1 et 55 % en poids, par rapport au poids de la solution aqueuse d'hydroxyde de métal alcalin.

5. Procédé selon l'une quelconque des revendications 1 à 4, dans lequel avant la distillation la solution d'hydroxyde de métal alcalin est mélangée avec l'aniline brute au moyen d'éléments mélangeurs statiques ou dynamiques.

6. Procédé selon l'une quelconque des revendications 1 à 5, dans lequel on effectue la distillation en un stade dans une colonne à courant latéral, les composants à bas point d'ébullition étant évacués par la tête, les composants à haut point d'ébullition étant évacués en tant que produit de pied et l'aniline purifiée étant déchargée en tant que courant latéral.

7. Procédé selon l'une quelconque des revendications 1 à 5, dans lequel on effectue la distillation en un stade dans une colonne à paroi de séparation, les composants à bas point d'ébullition étant évacués par la tête, les composants à haut point d'ébullition étant évacués en tant que produit de pied et l'aniline purifiée étant déchargée en tant que courant latéral.

8. Procédé selon l'une quelconque des revendications 6 et 7, dans lequel les vapeurs évacuées à la tête dans la colonne à courant latéral ou la colonne à paroi de séparation sont condensées dans une condensation en deux stades.

9. Procédé selon l'une quelconque des revendications 1 à 5, dans lequel la distillation s'effectue d'abord dans une colonne de distillation de composés à bas point d'ébullition, dans laquelle les composants à bas point d'ébullition et l'eau sont évacués à la tête de la colonne de distillation de composés à bas point d'ébullition et le mélange formé dans le pied, contenant de l'aniline et de l'hydroxyde de métal alcalin, est ensuite fractionné de nouveau dans une autre étape de distillation.

10. Procédé selon l'une quelconque des revendications 1 à 5, dans lequel les composants à bas point d'ébullition sont séparés de l'aniline brute par distillation avant l'addition de la solution d'hydroxyde de métal alcalin et dans lequel les composants à haut point d'ébullition sont séparés de l'aniline brute par distillation après l'addition de la solution d'hydroxyde de métal alcalin, de sorte qu'on obtient l'aniline purifiée en tant que produit de tête et les composants à haut point d'ébullition en tant que produit de pied.

11. Procédé selon la revendication 10, dans lequel les composants à haut point d'ébullition obtenus en tant que produit de pied sont ensuite concentrés dans une colonne de distillation.

12. Procédé pour la production de di- et polyamines de la série du diphénylméthane, dans lequel on produit de l'aniline conformément à un procédé selon la revendication 1 et ensuite on fait réagir l'aniline avec du formaldéhyde en présence d'un catalyseur acide, pour aboutir aux di- et polyamines.

13. Procédé pour la production de di- et polyisocyanates de la série du diphénylméthane, dans lequel on produit de l'aniline conformément à un procédé selon la revendication 1 et ensuite on fait réagir l'aniline avec du formaldéhyde en présence d'un catalyseur acide, pour aboutir à des di- et polyamines et ensuite on fait réagir les di- et polyamines avec du phosgène, pour aboutir aux di- et polyisocyanates.
